# EUROPEAN PATENT APPLICATION

(11) **EP 4 410 821 A1**
(43) Date of publication of application: **07.08.2024**
(21) Application number: 22876927.9
(22) Date of filing: 30.09.2022
(51) Int. Cl.: C07K 14/00, C07K 7/06, C07K 7/08, A61K 47/64, A61P 35/00

(54) **PEPTIDE FOR PENETRATING BLOOD-BRAIN BARRIER AND USE THEREOF**

(30) Priority: 01.10.2021 KR 20210130554
(71) Applicant: Seoul National University R&DB Foundation, Seoul 08826 (KR); Nibec Co., Ltd., Chungcheongbuk-do 27816 (KR)
(72) Inventor: PARK, Yoon Jeong, Seoul 08291 (KR); CHUNG, Chong-Pyoung, Seoul 05618 (KR); LEE, Jue-Yeon, Gwacheon-si Gyeonggi-do 13831 (KR); LEE, Dong Woo, Seoul 08652 (KR)
(74) Representative: Mewburn Ellis LLP
(86) International application number: PCT/KR2022/014716
(87) International publication number: WO 2023/055155

(57) **Abstract**

The present invention relates to a peptide for penetrating the blood-brain barrier and use thereof, and more particularly, a peptide for penetrating the blood-brain barrier and a complex in which the peptide is conjugated to a drug of antibody, protein or therapeutic nucleic acid, and use thereof for the treatment or prevention of brain diseases or brain tumors.

The peptide for penetrating the blood-brain barrier of the present invention passes through the cell membrane of brain endothelial cells with excellent efficiency, and the peptide-drug complex in which the peptide for penetrating the blood-brain barrier is combined with a drug such as an antibody, a protein or a therapeutic nucleic acid has the advantage of effectively delivering the drug into the brain parenchyma and maximizing the therapeutic effect of brain diseases and brain tumors.

## Description

### Technical Field

The present invention relates to a peptide for penetrating the blood-brain barrier and use thereof, and more specifically, a peptide for penetrating the blood-brain barrier, or a conjugate in which a drug, such as an antibody, a protein or a therapeutic nucleic acid, is conjugated to the peptide; and use thereof for the treatment or prevention of brain diseases or brain tumors.

### Related Art

The blood-brain barrier (BBB) is the most powerful biological barrier, a defense system that helps maintain the brain's complex physiology by selectively allowing only substances in the blood that are essential for maintaining brain function to pass through. The BBB is composed of capillaries formed from brain endothelial cells and the astrocytes and pericytes that surround them, each of which contributes to the strength of the BBB's unique biofilm. Unlike endothelial cells in other organs, brain endothelial cells have tight junctions that strongly inhibit the transcellular passage of 300 Da or more hydrophilic substances, which is known as physical barrier function. In addition, polarized endothelial cells have various types of multidrug efflux pumps, such as P-glycoprotein, MRP1, MRP4 and BCRP, which bind hydrophobic drugs and prevent drug penetration into brain tissue, which is called metabolic barrier function. Astrocytes extend their protrusions toward established brain capillaries to form astrocytic endfeet that cover most of the surface of the vessel, and it has recently been suggested that these endfeet play a pivotal role in maintaining the biological barrier by defending against harmful factors to the BBB.

Therefore, neurological drugs, such as drugs with large molecular weight that need to work inside the brain or drugs with small molecule weight with low brain penetration, cannot cross the blood-brain barrier (BBB).

Previous technologies have utilized the transcytosis pathway mediated by endogenous receptors expressed on the endothelium of brain capillaries, to cross the blood-brain barrier. Antibodies or peptides against transferrin receptors were designed based on the fact that many transferrin receptors are present on the surface of brain endothelial cells. Antibodies that bind to transferrin receptors cannot directly cross the cell membrane and can enter the brain endothelial cells by endocytosis. They have a limitation that, once entering into the cerebrovascular cells by endocytosis, they form endosomes, and if they cannot escape from the endosomes, they are transferred to the lysosome for degradation.

Brain endothelial cells also contain receptors for low density lipoprotein receptor (LDLR)-related proteins (LRPs), in addition to transferrin. The low density lipoprotein receptor-related proteins receptor endocytoses LDL particles, making them permeable to the BBB. These LRPs receptors do not undergo lysosomal translocation and degradation, and are favorable receptors for the delivery of drugs used to treat neurodegenerative diseases.

Previous studies have shown that IgG injected into the bloodstream permeates the CNS compartment at a very low rate (about 0.1%) [Felgenhauer, Klin. Wschr. 52: 1158-1164 (1974)], resulting in very limited pharmacokinetic effects within the CNS. Therefore, there is a need for delivery systems for neurological drugs that can cross the BBB and effectively transport the drug to the brain.

Accordingly, the inventors of the present invention have prepared a peptide for penetrating the blood-brain barrier (BBB) in which a cell-permeable peptide is linked to a transferrin receptor binding peptide or a low density lipoprotein receptor-related protein receptor binding peptide, and have found that the prepared peptide can directly cross the cell membrane of a brain endothelial cell without relying on endocytosis, and the peptide-drug complex for penetrating the blood-brain barrier in which a drug, such as an antibody, protein, or therapeutic nucleic acid, is conjugated to the peptide for penetrating the blood-brain barrier is effectively delivered to the brain parenchyma, and has the effect of treating brain tumors, and completed the present invention.

The above information in this Related Art is intended solely to enhance the understanding of the background of the present invention and may not include information that constitutes prior arts known to one having ordinary skill in the art.

### Summary of Invention

It is an object of the present invention to provide a peptide for penetrating the blood-brain barrier (BBB) to effectively deliver drugs for the treatment of brain diseases or brain tumors into the brain parenchyma.

Another object of the present invention is to provide a peptide-drug complex for penetrating the blood-brain barrier in which a drug is conjugated to the peptide for penetrating the blood-brain barrier.

Another object of the present invention is to provide a pharmaceutical composition for treating or preventing brain diseases or brain tumors, comprising the peptide-drug complex for penetrating the blood-brain barrier.

Another object of the present invention is to provide a method of preventing or treating brain diseases or brain tumors, comprising the step of administering the complex, use of the complex for preventing or treating brain diseases or brain tumors, and use of the complex for manufacturing a medicament for preventing or treating brain diseases or brain tumors.

To accomplish the above objectives, the present invention provides a peptide for penetrating the blood-brain barrier (BBB), comprising a brain endothelial cell surface protein-binding peptide and a cell-permeable peptide.

The present invention also provides a peptide-drug complex for penetrating the blood-brain barrier, wherein a drug is conjugated to the peptide for penetrating the blood-brain barrier.

The present invention also provides a nucleic acid encoding the complex.

The present invention also provides a recombinant vector into which the nucleic acid is introduced.

The present invention also provides a recombinant cell into which the nucleic acid or the recombinant vector is introduced.

The present invention also provides a method of producing a peptide-drug complex for penetrating the blood-brain barrier, comprising (a) culturing the recombinant cells to express the peptide-drug complex for penetrating the blood-brain barrier; and (b) recovering the expressed the peptide-drug complex for penetrating the blood-brain barrier.

The present invention also provides a pharmaceutical composition for treating or preventing brain diseases or brain tumors, comprising the peptide-drug complex for penetrating the blood-brain barrier.

The present invention also provides a method of preventing or treating brain diseases or brain tumors, comprising the step of administering the complex, use of the complex for preventing or treating brain diseases or brain tumors, and use of the complex for manufacturing a medicament for preventing or treating brain diseases or brain tumors.

### Brief Description of Drawing

FIG. 1 is a schematic of an antibody and a peptide-drug complex for penetrating the blood-brain barrier.
FIG. 2 is a schematic of an siRNA and a peptide-drug complex for penetrating the blood-brain barrier.
FIG. 3 shows SDS-PAGE and western blot results of a peptide-drug complex for penetrating the blood-brain barrier and an antibody expressed in pcDNA3.4-TOPO and purified.
FIG. 4a is an IVIS observation of a complex of a peptide for penetrating the blood-brain barrier and an antibody distributed in the brain, liver, lung, spleen, kidney and heart. FIG. 4b is a three-dimensional view of a complex of a peptide for penetrating the blood-brain barrier and an antibody distributed in the brain.
FIG. 5 is the concentration of a complex of a peptide for penetrating the blood-brain barrier and an antibody in the blood and brain.
FIG. 6a is an image of the bioluminescence measurement of U87MG-Luc cells on day 16. FIG. 6b is the results of measuring bioluminescence of U87MG-Luc cells for 16 days. FIG. 6c shows the distribution of the complex of the peptide for penetrating the blood-brain barrier and the antibody at the brain tumor site.
FIG. 7 shows the results of electrophoresis of the complex of a peptide for penetrating the blood-brain barrier and siRNA.
FIG. 8 shows the complex of siRNA with a peptide for penetrating the blood-brain barrier by transmission electron microscopy.
FIG. 9 shows the permeability results of a complex of a peptide for penetrating the blood-brain barrier in an in vitro BBB model and siRNA.
FIG. 10 shows the results of a complex of a peptide for penetrating the blood-brain barrier and siRNA permeabilized into the brain parenchyma.
FIG. 11 shows the results of measuring the mRNA levels of beta catenin reduced by a complex of a peptide for penetrating the blood-brain barrier and siRNA in the cerebral cortex, cerebellum, and midbrain as measured by qRT-PCR.

### Detailed Description of the Invention and Preferred Embodiments

Unless otherwise defined, all technical and scientific terms used herein have the same meaning as commonly understood by a person skilled in the art. In general, the nomenclature used herein is well known and in common use in the art.

In the present invention, a peptide capable of binding to transferrin receptors using phage display techniques instead of antibodies was designed to prevent the endosomal to lysosomal transfer of substances permeated into brain endothelial cells. This transferrin receptor binding peptide is characterized by binding to a different part of the transferrin receptor than the part where the transferrin binds to the transferrin receptor. Furthermore, low density lipoprotein receptor (LDLR)-related proteins (LRPs) receptor binding peptides have been devised.

By linking a transferrin receptor-binding peptide or low density lipoprotein receptor-related protein receptor binding peptide with a peptide with cell-penetrating function, the inventors of the subject invention have developed a peptide for penetrating the blood-brain barrier (BBB) that can directly penetrate the cell membrane of brain endothelial cells without relying on endocytosis, and named NIPEP-TPP-BBB shuttle (NIBEC Peptide-Target Tissue Penetrating Property-BBB shuttle).

In addition, drugs such as antibodies, proteins, or therapeutic nucleic acids were attached to the N-terminus of the peptide for penetrating the blood-brain barrier using gene expression method or chemical coupling method to deliver it to the brain parenchyma when intravenously injected into mice, and tumor cells were reduced when administered to animals with glioblastoma.

Thus, in one aspect, the present invention relates to a peptide for penetrating the blood-brain barrier (BBB) comprising a brain endothelial cell surface protein-binding peptide and a cell-permeable peptide.

In the present invention, the brain endothelial cell surface protein may be characterized as being a transferrin receptor or a low density lipoprotein receptor-related protein (LRP) receptor. Thus, the peptide for penetrating the blood-brain barrier may be characterized by a peptide capable of binding to a transferrin receptor or a peptide capable of binding to a low density lipoprotein receptor (LDLR)-related protein receptor is linked to a cell-permeable peptide.

In the present invention, the transferrin receptor binding peptide may be represented by an amino acid sequence of any one of SEQ ID NO: 1 to SEQ ID NO: 4.
SEQ ID NO: 1: VPALR
SEQ ID NO: 2: LRRERQSRLRRRERQSR
SEQ ID NO: 3: VPALRRERQSRLRRERQSR
SEQ ID NO: 4: HAIYPRH

In the present invention, the term "transferrin receptor" refers to a specific receptor on transferrin for the entry of iron into a cell, comprising a homodimer having polypeptide chains with a molecular weight of 95,000 Da, each chain having one molecule of transferrin in which iron ions are bound.

In the present invention, the term "transferrin" refers to a type of glycoprotein, an iron-transporting protein, wherein β-globulin binds to two molecules of divalent iron ion absorbed to serum, and iron required for cell proliferation or hemoglobin production is delivered, and 99% or more of the iron in serum is bound to transferrin.

In the present invention, the low density lipoprotein receptor-related protein receptor binding peptide may be represented by an amino acid sequence of SEQ ID NO: 5 or SEQ ID NO: 6.
SEQ ID NO: 5: YHFNGCEDPLCR
SEQ ID NO: 6: HPWCCGLRLDLR

In the present invention, the term "low density lipoprotein receptor-related protein receptor (LRP receptor)" refers to a cell membrane protein known to be highly expressed in brain endothelial cells, small intestinal epithelium, and the like, and functions to endocytose LDL particles and permeabilize the BBB.

In the the present invention, the term "low density lipoprotein receptor (LDLR)-related proteins (LRPs)" refers to glycoproteins of 600 kDa belonging to the LDL receptor gene family, which are expressed in a number of tissues, including hepatocytes, adipocytes, fibroblasts, macrophages and neurons of the central nervous system (Herz J., et al., EMBO Journal, 20, 4119-27 (1988)), with high concentrations in the liver and brain. The function of LRP in the central nervous system is particularly important in the cerebellum, cerebral cortex, hippocampus, and brainstem (Qian Z., et al., Nature, 361, 453-457 (1993)).

In the present invention, the cell-permeable peptide may be represented by an amino acid sequence of any one of SEQ ID NO: 7 to SEQ ID NO: 10.
SEQ ID NO: 7: HRRCNKNNKKR
SEQ ID NO: 8: HRRCNPNNKKR
SEQ ID NO: 9: VSRRRRRRRGGRRRR
SEQ ID NO: 10: GKCSTRGRKCCRRKK

In the present invention, the cell-permeable peptide may be characterized by being directly linked to the N-terminus, C-terminus, or both sides of the N-terminus and C-terminus of the transferrin receptor binding peptide or low density lipoprotein receptor-related protein receptor binding peptide, but is not limited thereto.

In the present invention, the peptide for penetrating the blood-brain barrier can be characterized by being represented by an amino acid sequence of any one of SEQ ID NO: 11 to SEQ ID NO: 58 (Table 1).

**[Table 1]**

| Amino acid sequence of a peptide for penetrating the blood-brain barrier | | | | | | |
|---|---|---|---|---|---|---|
| | VPALR (SEQ ID NO: 1) | LRRERQSR LRRRERQS R (SEQ ID NO: 2) | VPALRRER QSRLRRRE RQSR (SEQ ID NO: 3) | HAIYPRH (SEQ ID NO: 4) | YHFNGCE DPLCR (SEQ ID NO: 5) | HPWCCGL RLDLR (SEQ ID NO: 6) |
| HRRCNKN NKKR (SEQ ID NO: 7) | VPALR- HRRCNKN NKKKR (SEQ ID | LRRERQSR LRRRERQS R- HRRCNKN | VPALRRER QSRLRRRE RQSR- HRRCNKN | HAIYPRH- HRRCNKN NNKKKR (SEQ ID | YHFNGCE DPLCR- HRRCNKN NNKKKR | HPWCCGL RLDLR- HRRCNKN NNKKKR |
| | NO: 11) | NKKKR (SEQ ID NO: 19) | NKKKR (SEQ ID NO: 27) | NO: 35) | (SEQ ID NO: 43) | (SEQ ID NO: 51) |
| HRRCNPN NKKR (SEQ ID NO: 8) | VPALR- HRRCNPN NKKKR (SEQ ID NO: 12) | LRRERQSR LRRRERQS R- HRRCNPN NKKR (SEQ ID NO: 20) | VPALRRER QSRLRRER QSR- HRRCNPN KNKKR (SEQ ID NO: 28) | HAIYPRH- HRRCNPN NKKKR (SEQ ID NO: 36) | YHFNGCE DPLCR- HRRCNPN KNKKR (SEQ ID NO: 44) | HPWCCGL RLDLR- HRRCNPN KNKKR (SEQ ID NO: 52) |
| VSRRRRR RRGGRRR RR (SEQ ID NO: 9) | VPALR- VSRRRRR RRGGRRR RR (SEQ ID NO: 13) | LRRERQSR LRRRERQS R- VSRRRRR RRGGRRR RR (SEQ ID NO: 21) | VPALRRER QSRLRRRE RQSR- VSRRRRR RRGGRRR R (SEQ ID NO: 29) | HAIYPRH- VSRRRRR RRGGRRR RR (SEQ ID NO: 37) | YHFNGCE DPLCR- VSRRRRR RRGGRRR R (SEQ ID NO: 45) | HPWCCGL RLDLR- VSRRRRR RRGGRRR R (SEQ ID NO: 53) |
| GKCSTRG RKCCRRK K (SEQ ID NO: 10) | VPALR- GKCSTRG RKCCRRK K (SEQ ID NO: 14) | LRRERQSR LRRRERQS R- GKCSTRG RKCCRRK K (SEQ ID NO: 22) | VPALRRER QSRLRRER QSR- GKCSTRG RKCCRRK K (SEQ ID NO: 30) | HAIYPRH- GKCSTRG RKCCRRK K (SEQ ID NO: 38) | YHFNGCE DPLCR- GKCSTRG RKCCRRK K (SEQ ID NO: 46) | HPWCCGL RLDLR- GKCSTRG RKCCRRK KK (SEQ ID NO: 54) |
| HRRCNKN NKKR (SEQ ID | HRRCNKN NNKKKR- VPALR | HRRCNKN NKKKR- LRRERQSR | HRRCNKN NNKKKR- VPALRRER | HRRCNKN NNKKKR- HAIYPRH | HRRCNKN NNKKKR- YHFNGCE | HRRCNKN NKKKR- HPWCCGL |
| NO: 7) | (SEQ ID NO: 15) | LRRRERQS R (SEQ ID NO: 23) | QSRLRRRE RQSR (SEQ ID NO: 31) | (SEQ ID NO: 39) | DPLCR (SEQ ID NO: 47) | RLDLR (SEQ ID NO: 55) |
| HRRCNPN NKKR (SEQ ID NO: 8) | HRRCNPN NKKKR- VPALR (SEQ ID NO: 16) | HRRCNPN NKKKR- LRRERQSR LRRRERQS R (SEQ ID NO: 24) | HRRCNPN NKKKR- VPALRRER QSRLRRRE RQSR (SEQ ID NO: 32) | HRRCNPN NKKKR- HAIYPRH (SEQ ID NO: 40) | HRRCNPN KNKKR- YHFNGCE DPLCR (SEQ ID NO: 48) | HRRCNPN KNKKR- HPWCCGL RLDLR (SEQ ID NO: 56) |
| VSRRRRR RRGGRRR RR (SEQ ID NO: 9) | VSRRRRR RRGGRRR RR-VPALR (SEQ ID NO: 17) | VSRRRRR RRGGRRR R- LRRERQSR LRRRERQS R (SEQ ID NO: 25) | VSRRRRR RRGGRRR RR- VPALRRER QSRLRRRE RQSR (SEQ ID NO: 33) | VSRRRRR RRGGRRR RR- HAIYPRH (SEQ ID NO: 41) | VSRRRRR RRGGRRR RR- YHFNGCE DPLCR (SEQ ID NO: 49) | VSRRRRR RRGGRRR R- HPWCCGL RLDLR (SEQ ID NO: 57) |
| GKCSTRG RKCCRRK K (SEQ ID NO: 10) | GKCSTRG RKCCRRK KK-VPALR (SEQ ID NO: 18) | GKCSTRG RKCCRRK K- LRRERQSR LRRRERQS R (SEQ ID NO: 26) | GKCSTRG RKCCRRK K- VPALRRER QSRLRRRE RQSR (SEQ ID NO: 34) | GKCSTRG RKCCRRK K-HAIYPRH (SEQ ID NO: 42) | GKCSTRG RKCCRRK K- YHFNGCE DPLCR (SEQ ID NO: 50) | GKCSTRG RKCCRRK K- HPWCCGL RLDLR (SEQ ID NO: 58) |

In the present invention, the brain endothelial cell surface protein-binding peptide and cell-permeable peptide may be linked via a spacer comprising 2 to 10 amino acids G and 1 to 5 amino acids S.

In another aspect, the present invention relates to a peptide for penetrating the blood-brain barrier-drug complex, wherein a drug is conjugated to the peptide for penetrating the blood-brain barrier.

In the present invention, the peptide and drug for penetrating the blood-brain barrier are connected by a linker, but are not limited thereto.

In the present invention, the drug may be an antibody, a protein or a therapeutic nucleic acid, wherein the antibody may be an antibody that inhibits a protein that causes a brain disease, and the protein may be a growth factor associated with neurons, but is not limited thereto.

In the present invention, the protein may be selected from the group consisting of brain-derived neurotrophic factor (BDNF), nerve growth factor (NGF), neurotrophin-4/5, fibroblast growth factor (FGF)-2 and other FGFs, neurotrophin (NT)-3, erythropoietin (EPO), epidermal growth factor (EGF), transforming growth factor (TGF)-alpha, TGF-beta, vascular endothelial growth factor (VEGF), glial cell-derived neurotrophic factor (GDNF), neurturin, platelet-derived growth factor (PDGF), herregulin, neuregulin, artemisin, persepsin, interleukin, glial cell line-derived growth factor (GFR), granulocyte-colony stimulating factor (CSF), granulocyte-macrophage-CSF, and stem cell factor (SCF).

In the present invention, the antibody can be represented by an amino acid sequence of SEQ ID NO: 59 and has therapeutic efficacy in glioblastoma.

SEQ ID NO: 59:

MAWVWTLLFLMAAAQSIQAEWIKQRPGQGLEWIGVIHPGNGGTNYNENFKGKATLTA

DKSSSTAYMQLSSLTSDDSAVYFCASGNDGSYWGQGTTVTVSSGGGGSGKGGSGGGGSGG

GGGGGGSDIELTQSPSSLTVTAGEKVTMSCKSSQSLLNSGDQKIYLTWYQQKPGQPPKLLLIY

WASTRESGVPDRFTGSVSGTDFTLTISSVQAEDLAVYYCQNDYNYPYTFGGGTKCLEIKRGSE

NLYFQGGSGKPIPNPLLGLDSTGGSGGSHHHHHHHHHH

In the present invention, a peptide-drug complex for penetrating the blood-brain barrier in which an antibody is conjugated to the peptide for penetrating the blood-brain barrier can be prepared by a gene expression method or a chemical conjugation method, but the preparation method is not limited thereto.

For preparing a peptide-drug complex for penetrating the blood-brain barrier by gene expression method, a vector expressed together with (i) an antibody capable of inhibiting glioblastoma and (ii) a peptide for penetrating the blood-brain barrier can be utilized. In the present invention, the pET vector was used for expression in E. coli, and the pcDNA3.1-TOPO and pcDNA3.4-TOPO vectors were used for expression in mammalian cells.

In this case, a linker sequence may be further included between the antibody and the peptide for penetrating the blood-brain barrier for expression, and preferably, the linker may be, but is not limited to, CGGGG.

In the present invention, a therapeutic antibody can be expressed in a gene, purified, and chemically coupled to a peptide for penetrating the blood-brain barrier.

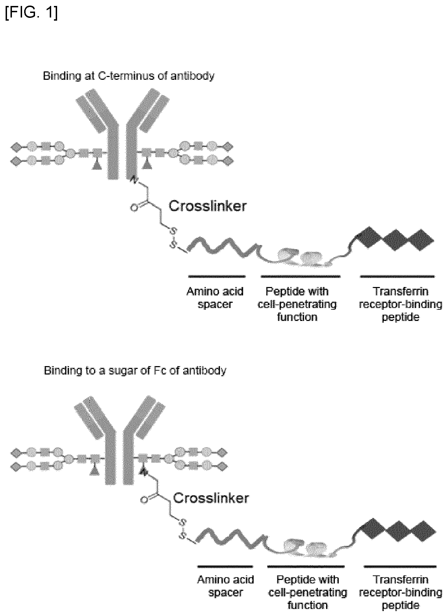

In the present invention, a peptide-drug complex for penetrating the blood-brain barrier of a therapeutic antibody and a peptide for penetrating the blood brain barrier, wherein a transferrin receptor selected from the group consisting of SEQ ID NO: 1 to SEQ ID NO: 4, or a low density lipoprotein receptor-related protein receptor binding peptide of SEQ ID NO: 5 or 6, and a cell-permeable peptide selected from the group consisting of SEQ ID NO: 7 to SEQ ID NO: 10 are chemically linked to a cysteine at the C-terminus of the antibody of SEQ ID NO: 59 or to a sugar in the Fc region. This is illustrated in the schematic of FIG. 1 showing the complex of an antibody and a peptide for penetrating the blood-brain barrier.

The peptide for penetrating the blood-brain barrier may be chemically linked to the antibody by linkers and crosslinkers. The linker may be any one that can provide space to form a functional structure. For example, the linker may be a peptidic linker of natural and/or synthetic origin. Peptidic linkers of natural and/or synthetic origin may be composed of amino acid chains of 1 to 50 amino acids, and may comprise repeating amino acid sequences of naturally occurring polypeptides, such as polypeptides with hinge functions. In another aspect, the peptidic linker amino acid sequence may be a synthetic linker amino acid sequence specified to have rich glycine, glutamine, and/or serine residues. These residues may be arranged in small repeating units of, for example, five or fewer amino acids, and the small repeating units may be repeated and arranged to form a multimer unit. At the amino- and/or carboxy-terminus of the multimer unit, six or fewer additional naturally occurring amino acids may be added. Other synthetic peptidic linkers may be of a single amino acid composition with 10 to 20 repeats, and no more than 6 additional naturally occurring amino acids at the amino- and/or carboxy-termini. Meanwhile, the linker may be a chemically modified form of the amino acid, for example, but not limited to, in the form of Fmoc-6-aminohexanoic acid conjugated with Fmoc-(9-Fluorenylmethoxycarbonyl) as a blocking group.

In some aspects according to the present invention, a sulfhydryl group of cysteine located at the N terminus of the peptide for penetrating the blood-brain barrier can be linked to an amine group of lysine in the exposed portion of the three-dimensional structure of SEQ ID NO: 59.

In this case, the crosslinkers that can be used may be, but is not limited to, 1,4-bis-maleimidobutane (BMB), 1,11-bis-maleimidotetraethyleneglycol (BM[PEO]4), 1-ethyl-3-[3-dimethyl aminopropyl] carbodiimide hydrochloride (EDC), succinimidyl-4-[N-maleimidomethylcyclohexane-1-carboxy-[6-amidocaproate]] (SMCC) and its sulfonated salt (sulfo-SMCC), succimidyl 6-[3-(2-pyridyldithio)-ropionamido] hexanoate](succimidyl 6-[3-(2-pyridyldithio)-ropionamido] hexanoate (SPDP) and its sulfonated salt (sulfo-SPDP), m-maleimidobenzoyl-N-hydroxysuccinimide ester (MBS) and its sulfonated salts (sulfo-MBS), or succimidyl[4-(p-maleimidophenyl) butyrate] (SMPB) and its sulfonated salts (sulfo-SMPB).

In the present invention, a method of preparing a peptide-drug complex for penetrating the blood-brain barrier using the above chemical coupling method comprises the following steps:
(a) activating a cysteine at the C-terminus of the therapeutic antibody or an amine group of lysine present in the exposed portion with a crosslinker;
(B) linking a peptide for penetrating the blood-brain barrier to the antibody activated by a crosslinker; and
(c) purifying a peptide-drug complex for penetrating the blood-brain barrier, wherein the antibody and a peptide for penetrating the blood-brain barrier.

In the present invention, the therapeutic nucleic acid may be selected from the group consisting of DNA, siRNA, miRNA and mRNA, and preferably the therapeutic nucleic acid has the function of inhibiting or increasing the expression of a target gene. The siRNA may have a base sequence complementary to a base sequence encoding an oncogenic protein, or a protein causing brain disease.

In one aspect of the present invention, expression of mutant KRAS is inhibited using siRNA against mutant KRAS, wherein the siRNA may be represented by SEQ ID NO: 60 or SEQ ID NO: 61.

KRAS siRNA sequence:
Sense-GUGCAAUGAAGGGACCAGUA (SEQ ID NO: 60);
Anti-sense-UACUGGUCCCUCAUUGCAC (SEQ ID NO: 61)

In the present invention, the siRNA may be in its natural form or a chemically modified form of siRNA.

The present invention may be characterized in that the therapeutic nucleic acid is reacted with a cationic peptide, cationic polymer, or RNA binding peptide to prepare a primary complex, and then the primary complex is reacted with an anionic amino acid or an anionic polymer; and the peptide for penetrating the blood-brain barrier linked with a spacer to form a nanoparticle.

In the present invention, the cationic peptide is represented by the amino acid sequence of SEQ ID NO: 9, the cationic polymer is poly-L-lysine, polyethylene imine or chitosan, and the RNA binding peptide is represented by the amino acid sequence of any one of SEQ ID NO: 62 to SEQ ID NO: 67, the anionic amino acid is glutamic acid or aspartic acid, the anionic polymer is heparin or hyaluronic acid, and the spacer is 5 to 10 glycines, but is not limited thereto.

To permeabilize the therapeutic nucleic acid across the blood brain barrier, an RNA binding peptide may be introduced in addition to the peptide for penetrating the blood-brain barrier, wherein the RNA binding peptide is represented by an amino acid sequence of any one of SEQ ID NO: 62 to SEQ ID NO: 67.
SEQ ID NO: 62: CPISQVHEIGIKRNMTVHFKVLREEGPAHMKNF
SEQ ID NO: 63: CPISQVHEIGIKR
SEQ ID NO: 64: CNMTVHFKVLREEG
SEQ ID NO: 65: CPAHMKNFITA
SEQ ID NO: 66: CIVTEGEGNGKKVSKKKRAAEKMLVEL
SEQ ID NO: 67: CEGNGKKVSKKRAA

The peptide of SEQ ID NO: 62 to SEQ ID NO: 67 can be used in conjunction with, but not limited to, the N-terminus of the complex of SEQ ID NO: 11 to SEQ ID NO: 58.

In the present invention, the complex of siRNA and a peptide for penetrating the blood-brain barrier may be characterized in that at least one transferrin receptor binding peptide selected from the group consisting of SEQ ID NO: 1 to SEQ ID NO: 4, or a low density lipoprotein receptor-related protein receptor binding peptide of SEQ ID NO: 5 or SEQ ID NO: 6; at least one cell-permeable peptide selected from the group consisting of SEQ ID NO: 7 to SEQ ID NO: 10; and an RNA binding peptide consisting of SEQ ID NO: 62 to SEQ ID NO: 67 and siRNA of SEQ ID NO: 60 or SEQ ID NO: 61 form a complex by self-assembly. The complex forms nano-sized particles, which may be characterized as having a size of 10-200 nm.

In the present invention, the complex of siRNA and a peptide for penetrating the blood-brain barrier can be prepared in two steps. 1) The siRNA is first reacted with a cationic peptide or cationic polymer. The cationic peptide can be a peptide of SEQ ID NO: 9, and the cationic polymer can be poly-L-lysine, polyethylene imine, or chitosan. 2) The next step is to conjugate the peptide for penetrating the blood-brain barrier to the anionic amino acid or anionic polymer. In this case, the complex of peptide for penetrating the blood-brain barrier, spacer and anionic amino acid may be represented by an amino acid sequence of any one of SEQ ID NO: 68 to SEQ ID NO: 85.

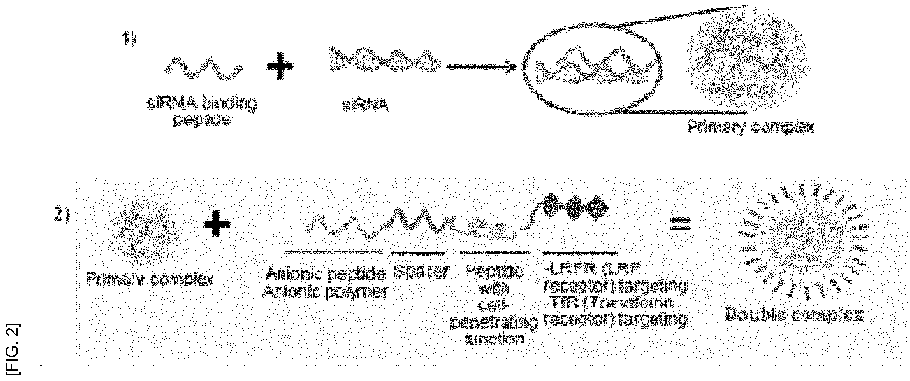

FIG. 2 is a schematic illustration of a complex of siRNA and a peptide for penetrating the blood-brain barrier. FIG. 2 is an exemplary illustration where the therapeutic nucleic acid is an siRNA, and where the siRNA is DNA, miRNA, or mRNA, the primary complex can be prepared by reacting with a DNA, miRNA, or mRNA binding peptide. The complex of a peptide for penetrating the blood-brain barrier and a therapeutic nucleic acid may comprise an anionic amino acid consisting of 5 to 10 glutamic acids or aspartic acids, a spacer consisting of 5 to 10 glycines, a cell-permeable functional peptide selected from the group consisting of SEQ ID NO: 7 to SEQ ID NO: 10, and a peptide conjugated with a brain endothelial cell surface protein-binding peptide selected from the group consisting of SEQ ID NO: 1 to SEQ ID NO: 6.
SEQ ID NO: 68: EEEGEEEGE-GGGGG-HRRCNKNNKKR-VPALR
SEQ ID NO: 69: EEEGEEEGE-GGGGG-HRRCNPNNKKR-GGGGG-VPALR
SEQ ID NO: 70: EEEGEEEGE-GGGGG-VSRRRRRRGGRRRR-GGGGG-VPALR
SEQ ID NO: 71: EEEGEEEGE-GGGGG-GKCSTRGRKCCRRKK-GGGGG-VPALR
SEQ ID NO: 72: EEEGEEEGE-GGGGG-HRRCNKNNKKR-LRRERQSRLRRERQSR
SEQ ID NO: 73: EEEGEEEGE-GGGGG-HRRCNPNNKKR-LRRERQSRLRRERQSR

SEQ ID NO: 74: EEEGEEEGE-GGGGG-VSRRRRRRGGRRRR-GGGGG-

LRRERQSRLRRERQSR

SEQ ID NO: 75: EEEGEEEGE-GGGGG-GKCSTRGRKCCRRKK-
LRRERQSRLRRERQSR

SEQ ID NO: 76: EEEGEEEGE-GGGGG-HRRCNKNNKKR-
VPALRRERQSRLRRERQSR

SEQ ID NO: 77: EEEGEEEGE-GGGGG-HRRCNPNNKKR-
VPALRRERQSRLRRERQSR

SEQ ID NO: 78: EEEGEEEGE-GGGGG-VSRRRRRRGGRRRR-

VPALRRERQSRLRRERQSR

SEQ ID NO: 79: EEEGEEEGE-GGGGG-GKCSTRGRKCCRRKK-
VPALRRERQSRLRRERQSR

SEQ ID NO: 80: EEEGEEEGE-GGGGG-HRRCNKNNKKR-HAIYPRH
SEQ ID NO: 81: EEEGEEEGE-GGGGG-HRRCNPNNKKR-HAIYPRH
SEQ ID NO: 82: EEEGEEEGE-GGGGG-VSRRRRRRGGRRRR-HAIYPRH
SEQ ID NO: 83: EEEGEEEGE-GGGGG-GKCSTRGRKCCRRKK-HAIYPRH

SEQ ID NO: 84: EEEGEEEGE-GGGGG-VSRRRRRRGGRRRR-GGGGG-

YHFNGCEDPLCR

SEQ ID NO: 85: EEEGEEEGE-GGGGG-VSRRRRRRGGRRRR-GGGGG-
HPWCCGLRLDLR

In addition, anionic polymers such as heparin or hyaluronic acid can be used instead of the anionic amino acids glutamic acid or aspartic acid.

By self-assembly, siRNA is first reacted with a peptide of SEQ ID NO: 9, and then a peptide for penetrating the blood-brain barrier is combined, and the complex forms nano-sized particles having a size of 10-200 nm.

In the present invention, a method of forming a complex of therapeutic nucleic acids, including siRNA, by the self-assembly comprises the following steps:
(a) first reacting a cationic peptide, cationic polymer, or RNA binding peptide with a therapeutic nucleic acid;
(b) reacting with the peptide for penetrating the blood-brain barrier introduced with an anionic peptide; and
(c) forming nanoparticles by self-assembly.

In another aspect, the present invention relates to a nucleic acid encoding the peptide-drug complex for penetrating the blood-brain barrier.

In another aspect, the present invention relates to a recombinant vector in which the nucleic acid is introduced.

In the present invention, the vector may be, but is not limited to, a pET vector, pcDNA 3.4, pcDNA3.1, pcDNA3.1-TOPO, pcDNA3.4-TOPO, or pSecTag vector, and preferably a pET vector or a pcDNA3.1-TOPO, pcDNA3.4-TOPO vector.

In another aspect, the present invention relates to a recombinant cell into which the nucleic acid or the recombinant vector is introduced.

In the present invention, the recombinant cell may be E. coli or mammalian cells, but is not limited thereto.

In the present invention, the mammalian cell line can be, but is not limited to, Chinese hamster ovarian cell line (CHO), human embryonic kidney cell line (HEK293), and human embryonic kidney cell line (HEK293).

In another aspect, the present invention relates to a method of preparing the peptide-drug complex for penetrating the blood-brain barrier, comprising the following steps:
(a) culturing the recombinant cell to express the peptide-drug complex for penetrating the blood-brain barrier; and
(b) recovering the expressed peptide-drug complex for penetrating the blood-brain barrier.

Antibodies that inhibit glioblastoma or siRNAs that inhibit mutations, which are representative antibodies that inhibit brain diseases, do not have a significant therapeutic effect because they are unable to penetrate the blood-brain barrier. On the other hand, in other embodiments of the present invention, when the brain delivery effect or tumor proliferation inhibition effect of antibodies or siRNAs conjugated with peptides that can penetrate the blood-brain barrier was checked, both in vitro and in animal experiments, it was found that the proliferation of the corresponding tumor cells was significantly inhibited.

Accordingly, in another aspect, the present invention relates to a pharmaceutical composition for treating or preventing brain diseases or brain tumors comprising the peptide-drug complex for penetrating the blood-brain barrier.

In another aspect, the present invention relates to a method of preventing or treating brain diseases or brain tumors, comprising the step of administering a peptide-drug complex for penetrating the blood-brain barrier.

In another aspect, the present invention relates to the use of the peptide-drug complex for penetrating the blood-brain barrier for the prevention or treatment of brain diseases or brain tumors.

In another aspect, the present invention relates to the use of the peptide-drug complex for penetrating the blood-brain barrier for the preparation of an agent for the prevention or treatment of brain diseases or brain tumors.

In the present invention, antibodies or therapeutic nucleic acid substances that can be used to treat other brain diseases in addition to brain tumor inhibition can be combined with peptides for penetrating the blood-brain barrier.

In the present invention, the brain disease may be selected from the group consisting of encephalitis, Parkinson's disease, epilepsy, Huntington's disease, Alzheimer's disease, Lou Gehrig's disease, Pick's disease, epilepsy, Creutzfeldt-Jakob disease, progressive supranuclear palsy, spinocerebellar degeneration, cerebellar atrophy, multiple sclerosis, senile dementia caused by the loss of nerve cells, stroke, amnesia, depression, and post-traumatic stress disorder.

In the present invention, the brain tumor may be selected from the group consisting of glioma, glioblastoma multiforme, meningioma, astrocytoma, acoustic neuroma, chondroma, rare glioblastoma, medulloblastoma, ganglioneuroma, schwannoma, neurofibroma, neuroblastoma, and epidural, intramedullary or intrathecal tumors.

In the present invention, a single dose of the peptide-drug complex for penetrating the blood-brain barrier may be 1 µg/kg to 100 mg/kg, preferably 5 µg/kg to 50 mg/kg, and may be characterized by administration once daily or 1-3 times per week, but the dose and dosing interval are not limited thereto.

In the present invention, the pharmaceutical composition may be formulated in any one of the dosage forms selected from the group consisting of, but not limited to, liquids (e.g., for injection) such as injectables, oral preparations, aqueous solutions or emulsions, capsules, granules, tablets, and mucosal delivery agents. These formulations may be prepared by conventional methods used in the art for formulation or by methods disclosed in Remington's Pharmaceutical Science (latest edition), Mack Publishing Company, Easton PA, and may be formulated in various formulations for different conditions or based on ingredients.

In the present invention, the pharmaceutical composition of the present invention may further comprise one or more pharmaceutically acceptable carriers in addition to the complex of antibody or siRNA conjugated to the peptide for penetrating the blood-brain barrier. The pharmaceutically acceptable carrier may be one or more selected from the group consisting of, but not limited to, saline, sterile water, Ringer's solution, buffered saline, dextrose solution, maltodextrin solution, glycerol, and ethanol.

The pharmaceutical composition of the present invention may further contain pharmaceutically acceptable auxiliaries as required. The adjuvant may be one or more selected from the group consisting of, but not limited to, excipients, diluents, dispersants, buffers, antimicrobial preservatives, bacteriostatic agents, surfactants, binders, lubricants, antioxidants, thickeners, and viscosity modifiers.

The pharmaceutical composition according to the present invention may be administered orally or parenterally (e.g., intravenously, subcutaneously, intraperitoneally or topically), depending on the intended method, and the dosage may be varied according to the expert's opinion, depending on the patient's weight, age, gender, health status, diet, time of administration, method of administration, excretion rate, and severity of the disease.

The present invention is described in more detail by providing the following examples. These examples are intended solely to illustrate the present invention, and it would be apparent to one of ordinary skill in the art that the scope of the present invention is not to be construed as limited by these examples.

### Example 1: Synthesis of a peptide for penetrating the blood-brain barrier

Amino acids and reagents required for synthesis were purchased from GL biochem and Sigma-Aldirich. The peptide was synthesized by F-moc solid-phase chemical synthesis from the C-terminus using the peptide synthesis method. That is, Rink resin with Fmoc-(9-Fluorenylmethoxycarbonyl) as the blocking group (0.075 mmol/g, 100 ~ 200 mesh, 1% DVB crosslinking) was synthesized by adding 50 mg of rink resin in the synthesizer, followed by swelling of the resin with DMF, and then using a 20% piperidine/DMF solution for removal of the Fmoc-group. Starting from the C-terminus, in sequence, 0.5 M amino acid solution (solvent: dimethylformamide, DMF), 1.0 M DIPEA (solvent: dimethylformamide & n-methylpyrrolidone, DMF & NMP), and 0.5 M HBTU (solvent: dimethylformamide, DMF) were added in 5, 10, and 5 equivalents, respectively, and reacted under a nitrogen stream for 1-2 hours. After each of the above deprotection and coupling steps, washing was conducted twice with DMF and isopropanol. Deprotection was also performed after coupling the last amino acid to remove the Fmoc-group. After removing the F-moc protecting group of the last amino acid at the N-terminus, the synthesis proceeded by adding DIPEA (10 equivalents), HBTU (5 equivalents), and palmitic acid (5 equivalents) in the presence of DMF. The reaction proceeded until a negative result was obtained using the Kaiser test solution for measuring the reaction time, and after the end of the reaction, the resin was washed with DMF and MeOH and dried in a vacuum oven. Trifluoroacetic acid (TFA) cleavage cocktail was added at a ratio of 20 ml per gram of resin and shaken for 3 hours to separate the resin and the peptide dissolved cocktail by filtering. The filtered solution was removed using a rotary evaporator and then cold ether was added or an excess of cold ether was added directly to the TFA cocktail solution in which the peptide was dissolved to crystallize the peptide into a solid phase, and then separated by centrifugation. The TFA cocktail was then completely removed by washing with ether several times and centrifugation. The resulting peptides were dissolved in distilled water and lyophilized. After lyophilization, they were separated and purified by high-performance liquid chromatography (Shimadzu, Japan). The analysis was performed using a 4.6 mm diameter C₁₈ column with 0.1% TFA/H₂O and 0.092% TFA/acetonitrile at a flow rate of 1 ml/min with a gradient of 0 to 60% for 30 min, and the wavelength of the UV detector was 220 nm. Purification was performed using a 2.2 cm diameter column at a flow rate of 20 ml/min with the same solvent and detection wavelength. The molecular weight of the purified peptide was confirmed by mass spectrometry.

1 mg of Alexa Fluor^{™} 680 NHS Ester (=Cy5.5-NHS ester, Invitrogen, A37567) and 2 mg of the synthesized peptide were dissolved in 0.1 ml of DMF. 0.05 ml pyridine was added and the reaction was performed on a 50°C heat block for 15 min. 0.7 ml ethyl acetate was added to form a precipitate, and then centrifuged at 10,000 rpm for 2 minutes. The supernatant was removed, washed twice with 0.7 ml of ethyl acetate in the same way, and dried in air.

### Example 2: Preparation of a complex of peptide and antibody for penetrating the blood brain barrier

The linker CGGGG was introduced at the N-terminus of the peptide for penetrating the blood-brain barrier (SEQ ID NO: 41) prepared using the method of Example 1, and the antibody of SEQ ID NO: 59 was chemically conjugated. 1.02 mg of antibody of SEQ ID NO: 59 was dissolved in 1 mL of PBS buffer (pH 8.3). 2.12 mg of SPDP (succinimidyl 3-(2-pyridyldithio)propionate, ThermoFisher, 21857) was dissolved in 120 µL DMSO (Dimethyl sulfoxide). 40 µL of SMCC solution was added thereto, and the reaction was repeated three times by shading for 1 hour. At the end of the reaction, desalting was performed using a PD-10 Desalting Column (GE healthcare) and 3.5 mL of pyridyldithiol activated antibody was obtained. 2.5 mg of SEQ ID NO: 42 solution was mixed in 300 µL of tertiary purified water and adjusted to pH 8.3 using 1 M tris buffer (pH=9). After shading, the reaction was performed overnight with mixing at 4°C. The antibody conjugated with a peptide for penetrating the blood brain barrier was purified by FPLC (Akta Pure, GE healthcare) using a heparin column (HiTrap, GE Healthcare).

For animal cell expression, the gene sequence, wherein the peptide for penetrating the blood brain barrier of SEQ ID NO: 15 to SEQ ID NO: 18 was conjugated to the C term of the antibody of SEQ ID NO: 59 in the pcDNA3.4-TOPO vector, was synthesized.
SEQ ID NO: 59-SEQ ID NO: 15 
MAWVWTLLFLMAAAQSIQAEWIKQRPGQGLEWIGVIHPGNGGTNYNENFKGKATLTA
DKSSSTAYMQLSSLTSDDSAVYFCASGNDGSYWGQGTTVTVSSGGGGSGKGGSGGGGSGG
GGSDIELTQSPSSLTVTAGEKVTMSCKSSQSLLNSGDQKIYLTWYQQKPGQPPKLLIYWASTR
ESGVPDRFTGSVSGTDFTLTISSVQAEDLAVYYCQNDYNYPYTFGGGTKCHRRCNKNNKKR-
VPALRLEIKRGSENLYFQGGSGKPIPNPLLGLDSTGGSGGSHHHHHH
SEQ ID NO: 59-SEQ ID NO: 16 
MAWVWTLLFLMAAAQSIQAEWIKQRPGQGLEWIGVIHPGNGGTNYNENFKGKATLTA
DKSSSTAYMQLSSLTSDDSAVYFCASGNDGSYWGQGTTVTVSSGGGGSGKGGSGGGGSGG
GGSDIELTQSPSSLTVTAGEKVTMSCKSSQSLLNSGDQKIYLTWYQQKPGQPPKLLIYWASTR
ESGVPDRFTGSVSGTDFTLTISSVQAEDLAVYYCQNDYNYPYTFGGGTKCHRRCNPNNKKR-
VPALRLEIKRGSENLYFQGGSGKPIPNPLLGLDSTGGSGGSHHHHHH
SEQ ID NO: 59-SEQ ID NO: 17 
MAWVWTLLFLMAAAQSIQAEWIKQRPGQGLEWIGVIHPGNGGTNYNENFKGKATLTA
DKSSSTAYMQLSSLTSDDSAVYFCASGNDGSYWGQGTTVTVSSGGGGSGKGGSGGGGSGG
GGSDIELTQSPSSLTVTAGEKVTMSCKSSQSLLNSGDQKIYLTWYQQKPGQPPKLLIYWASTR
ESGVPDRFTGSVSGTDFTLTISSVQAEDLAVYYCQNDYNYPYTFGGGTKCVSRRRRRRGGRR
RR-VPALRLEIKRGSENLYFQGGSGKPIPNPLLGLDSTGGSGGSHHHHHH
SEQ ID NO: 59-SEQ ID NO: 18

MAWVWTLLFLMAAAQSIQAEWIKQRPGQGLEWIGVIHPGNGGTNYNENFKGKATLTA
DKSSSTAYMQLSSLTSDDSAVYFCASGNDGSYWGQGTTVTVSSGGGGSGKGGSGGGGSGG
GGSDIELTQSPSSLTVTAGEKVTMSCKSSQSLLNSGDQKIYLTWYQQKPGQPPKLLIYWASTR
ESGVPDRFTGSVSGTDFTLTISSVQAEDLAVYYCQNDYNYPYTFGGGTKCGKCSTRGRKCCR
RKK-VPALRLEIKRGSENLYFQGGSGKPIPNPLLGLDSTGGSGGSHHHHHH

By cutting pcDNA3.1-TOPO or pcDNA3.4-TOPO with EcoR I and BamH I, a KRAS mutant scFv gene fragment with 900-960 bp was recovered by agarose electro elution. To insert it into a pcDNA3.1-TOPO or pcDNA3.4-TOPO vector, the gene was amplified by PCR, and BamH I and EcoR I restriction enzyme sites were synthesized at the same time. The respective truncated vectors and inserts were mixed in a ratio of 4 µl to 4 µl, respectively, and reacted for 16-18 hours at 4°C using T4 DNA ligase in ligation buffer solution composed of Tris-HCl 500 mM, MgCl₂ 100 mM, DTT 200 mM, and ATP 10 mM.

The prepared vector was transfected into the Chinese hamster ovary cell line ExpiCHO-S. The Chinese hamster ovary cell line ExpiCHO-S was obtained from Thermo Fisher Scientific (USA) and cultured in ExpiCHO Expression medium medium (GIBCO, USA). The ExpiCHO-S cell line (3×10⁸ cells) was then placed in 50 mL of culture medium in 250 mL disposable Erlenmeyer flasks. 2 mL of Opti-MEM containing 50 µg of plasmid was mixed with 160 µL of ExpiFectamine CHO Reagent (Gibco, Cat # A20130) diluted in 1.84 mL of Opti-MEM and it was stop-reacted for 5 minutes at room temperature, then evenly spotted onto the prepared Chinese hamster ovary cells, and incubated in 8% CO₂ thermostat at 37°C, for 18 hours, and then 300 µL of ExpiCHO enhancer and 12 mL of ExpiCHO Feed were added and incubated for 5 days. Six days after transfection, the culture medium was collected, sterilized, and purified using FPLC. Columns were separately purified using nickel chromatography (HisTrap^{™} excel, GE healthcare), pre-wetting the nickel column with buffer at [20 mM sodium phosphate, 0.5 M NaCl, pH 7.4] to equalize it, and then applying the medium. After washing with the buffer containing 25 mM of imidazole, proteins were separated by increasing the imidazole to 125 mM. The isolated proteins were pooled and stored by desalting to remove imidazole.

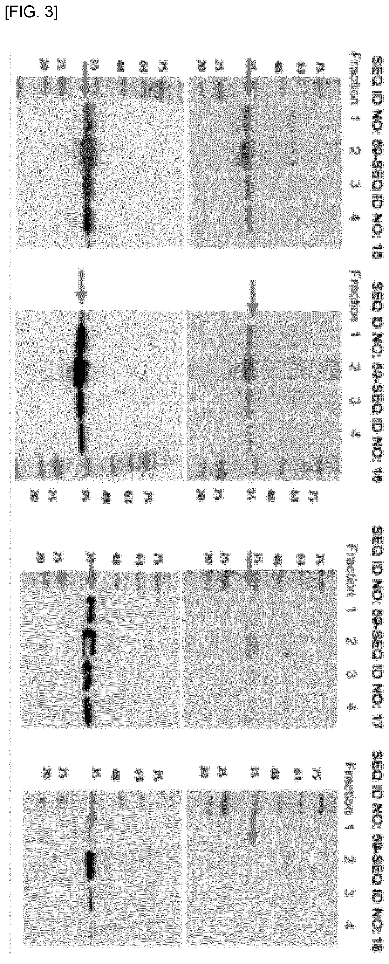

FIG. 3 shows the results of SDS-PAGE and western blot of the peptide for penetrating the blood-brain barrier and antibody expressed in pcDNA3.4-TOPO and purified. The arrow signs indicate the expressed complexes.

### Experimental Example 1: Confirmation of brain delivery of a complex of peptide for penetrating the blood-brain barrier and antibody in normal animals

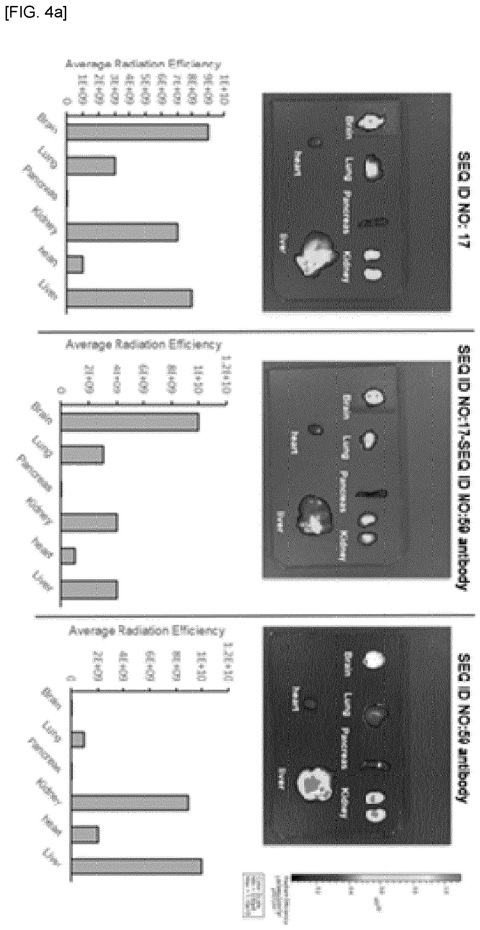

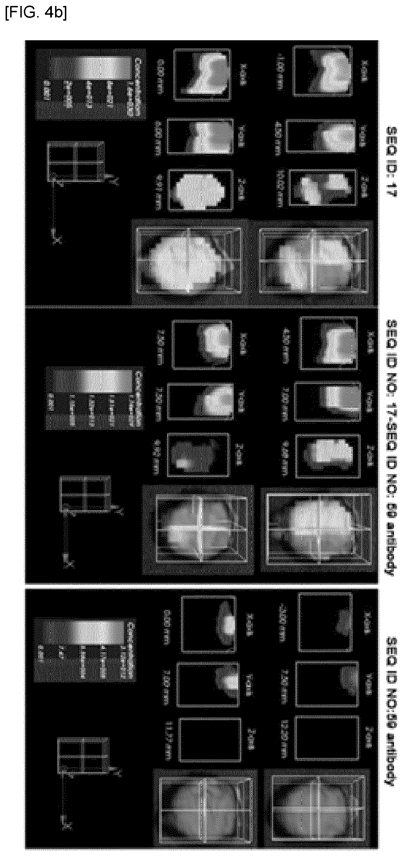

Cy5.5 labeled SEQ ID NO: 17 peptide for penetrating the blood-brain barrier, Cy5.5 labeled SEQ ID NO: 59 antibody, and the complex of Cy5.5-labeled SEQ ID NO: 17 peptide for penetrating the blood-brain barrier and SEQ ID NO: 59 antibody were injected intravenously at 4 mg/kg each to hairless mouse (SKH-1, female, 6 weeks old, OrientBio), and fluorescence in each organ (brain, liver, lung, spleen, kidney, heart) was measured at excitation: 685 nm and emission: 706 nm using IVIS (Perkin Elmer). In addition, three-dimensional images of the brain alone were measured using Optix MX3 (ART Advanced Research Technologies Inc.). FIG. 4 shows the results of the brain delivery of the peptide-antibody complex for penetrating the blood-brain barrier in animals.

As shown in FIG. 4(a), fluorescence could be observed in the brain upon injection of the Cy5.5-labeled SEQ ID NO: 17 peptide for penetrating the blood-brain barrier, and the complx of Cy5.5-labeled SEQ ID NO: 17 peptide for penetrating the blood-brain barrier and SEQ ID NO: 59 antibody. However, when administering the Cy5.5-labeled SEQ ID NO: 59 antibody, fluorescence was not observed in the brain. Furthermore, as shown in the three-dimensional image in FIG. 4(b), when the Cy5.5-labeled SEQ ID NO: 17 peptide for penetrating the blood-brain barrier and the complex of Cy5.5-labeled SEQ ID NO: 17 peptide for penetrating the blood-brain barrier and SEQ ID NO: 59 antibody were injected, the extent of penetration into the brain was large. However, the SEQ ID NO: 59 antibody was observed to a lesser extent inside the brain.

### Experimental Example 2: Determination of concentration in the blood and brain tissue of a complex of peptide for penetrating the blood-brain barrier and antibody in normal animals

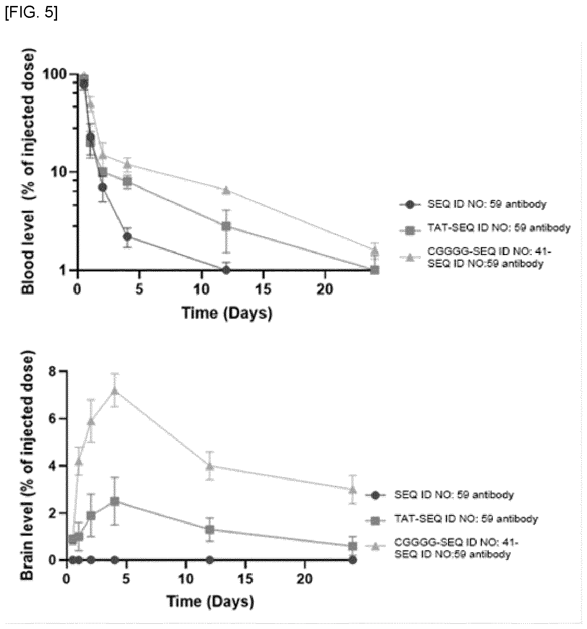

The control cell permeability peptide (TAT) and antibody of SEQ ID NO: 59 were prepared in the same manner as the expression method in Example 2 and labeled with cy5.5. The complex of the peptide for penetrating the blood-brain barrier of SEQ ID NO: 41 in which CGGGG was introduced in N term and the antibody of SEQ ID NO: 59 prepared in Example 2 was labeled labeled with Cy5.5. The antibody of SEQ ID NO: 59 was labeled with Cy5.5 and injected intravenously into hairless mouse (SKH-1, female, 6 weeks old, OrientBio) at 10 mg/kg each. Concentrations in brain and blood were measured at excitation: 685 nm and emission: 706 nm using IVIS (Perkin Elmer) until day 24, and the results are shown in FIG. 5.

When administered alone, the antibody was cleared from the blood within 12 days. Both the antibody linked to TAT and the antibody linked to the peptide for penetrating the blood-brain barrier of SEQ ID NO: 41 were retained up to 24 days. However, the blood levels of the complex of the peptide for penetrating the blood-brain barrier of SEQ ID NO: 41 and the antibody were higher.

In addition, no fluorescence was measured in brain tissue when the antibody was administered alone. With the TAT-linked antibody, 2.4% of the initial dose of fluorescence was measured in brain tissue 4 days after intravenous injection. The complex of the peptide for penetrating the blood-brain barrier of SEQ ID NO: 41 and the antibody of SEQ ID NO: 59 was measured approximately 7% of the initial dose in brain tissue 4 days after intravenous injection. This demonstrates that the complex of the peptide for penetrating the blood-brain barrier of SEQ ID NO: 41 and the antibody of SEQ ID NO: 59 are more efficient in brain delivery.

### Experimental Example 3: Confirmation of the effectiveness of a brain tumor treatment using a brain tumor animal model

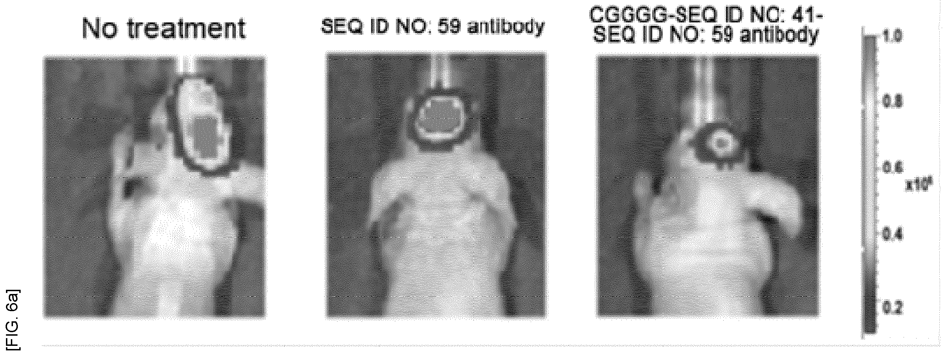

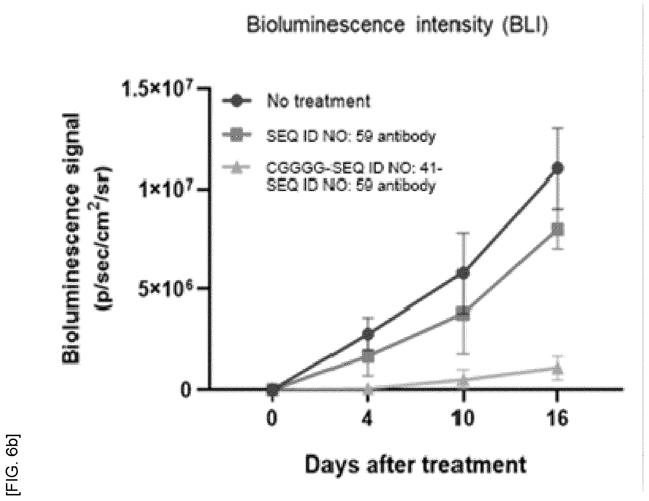

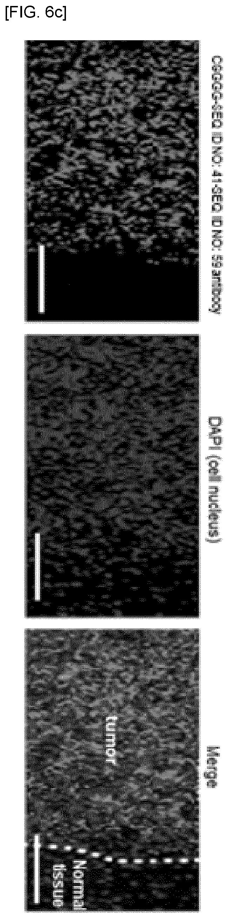

Using a brain tumor animal model, the ability of the complex of the peptide for penetrating the blood-brain barrier and antibody to penetrate the brain, inhibit brain tumors, and be distributed in the brain parenchyma was measured. Specifically, U87MG-Luc 2 (ATCC, HTB-14-LUC2^{™}) cells, a luciferase-expressing U87MG glioblastoma, were cultured in DMEM medium supplemented with 10% FBS and 1% antibiotics. Nude 5- to 7-week-old mouse (Orient Bio) was anesthetized with isoflurane and immobilized using ear bars inside a stereotaxic instrument. After incising the scalp at the surgical site, a hole was made using a sterile drill 2 mm to the right to and 2 mm under the bregma. Then, 1.0×10⁵ /10µL U87MG-Luc-2 cells were injected through the above hole with a 26G Hamilton syringe (1µl/min), and after injection, the hole was closed with bone wax and sutured to close the scalp. The proliferation of transplanted glioblastoma was obtained by injecting D-luciferin potassium (15 mg/mL dissolved in PBS, sigma), a substrate of luciferase, at 150 mg/kg, to a mouse, and sacrificing it after 10 min, and measuring the bioluminescence using IVIS (Perkin Elmer), and the results are shown in FIG. 6.

Starting 4 days after glioblastoma transplantation, 1 mg/kg of the complex of CGGGG-SEQ ID NO: 41 peptide for penetrating the blood-brain barrier and SEQ ID NO: 59 antibody and 1 mg/kg SEQ ID NO: 59 antibody were injected intravenously. The administration interval between doses was every 3 days for a total of 4 doses for 16 days. FIG. 6 shows the results of observing the penetration of the complex of the peptide for penetrating the blood-brain barrier and the antibody into the brain, inhibition of brain tumors, and distribution in the brain parenchyma in an animal model of brain tumors. FIG. 6(a) is the photo of an IVIS observation of bioluminescence of U87MG-Luc cells on day 16. FIG. 6(b) shows the bioluminescence of U87MG-Luc cells measured by IVIS for 4, 10, and 16 days. After 16 days of bioluminescence observation, the bioluminescence continued to increase in the glioblastoma transplanted and untreated group. This indicates that many of the transplanted brain tumor cells survived. When the antibody alone was administered, many brain tumor cells survived, but when the complex of CGGGG- SEQ ID NO: 41 peptide for penetrating the blood-brain barrier and SEQ ID NO: 59 antibody was injected, the bioluminescence caused by brain tumor cells decreased.

The complex of a peptide for penetrating the blood-brain barrier of CGGGG-SEQ ID NO: 41 labeled with cy5.5 and a SEQ ID NO: 59 antibody was administered to the same animal model 8 hours before sacrifice, and the brain was harvested. After frozen sectioning, the distribution of fluorescence was observed by confocal microscopy. In FIG. 6(c), the complex of the peptide for penetrating the blood-brain barrier of CGGGG-SEQ ID NO: 41 labeled with cy5.5 and the SEQ ID NO: 59 antibody was found to be distributed in the area where the brain tumor occurred.

### Example 3: Preparation of a complex of the peptide for penetrating the blood-brain barrier and RNA binding peptide with siRNA

Nanoparticles were prepared by self-assembly by mixing siRNA of SEQ ID NO: 60 or 61, RNA binding peptide of SEQ ID NO: 62, and peptide for penetrating the blood-brain barrier of SEQ ID NO: 40 in a certain ratio. The RNA binding peptide and the peptide for penetrating the blood brain barrier were dissolved in RNase, DNase Free & PCR certified Water (Tech&lnnovation, BWA-8000) at a concentration of 5 µg/µL. siRNA was dissolved in RNase, DNase Free & PCR certified Water (Tech&lnnovation, BWA-8000) at a concentration of 1 µg/µL. The mixing ratio of siRNA solution and peptide for penetrating the blood-brain barrier was 1:10 to 1:20, and 10 µL of siRNA was mixed with 12 µL (1:10) of peptide solution for penetrating the blood-brain barrier solution in 2 µL increments, and left for at least 30 min. Peptide for penetrating the blood-brain barrier of SEQ ID NO: 63-SEQ ID NO: 40, SEQ ID NO: 64-SEQ ID NO: 40, SEQ ID NO: 65-SEQ ID NO: 40, SEQ ID NO: 66-SEQ ID NO: 40, and SEQ ID NO: 67-SEQ ID NO: 40 also form the complex with siRNA of SEQ ID NO: 60 or SEQ ID NO: 61 in the same manner as above.

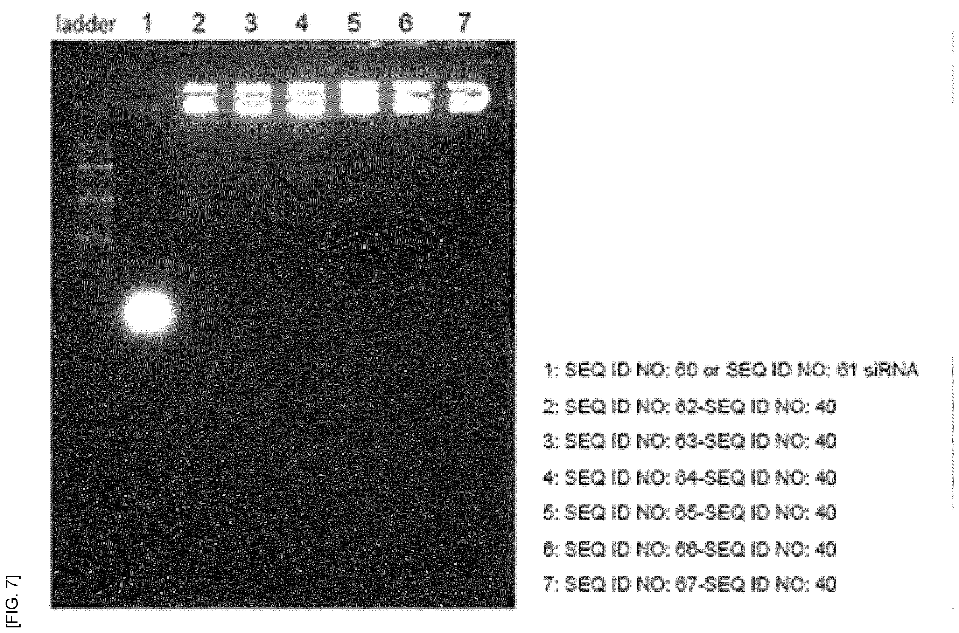

FIG. 7 shows the results of electrophoresis of the complex of siRNA and a peptide for penetrating the blood-brain barrier. The siRNA alone migrates downward, but the complex does not.

### Example 4: Preparation of a complex of peptide for penetrating the blood-brain barrier, cationic peptide and siRNA

### Example 4-1: Preparation of a complex of a peptide for penetrating the blood brain barrier comprising a transferrin receptor binding peptide and KRAS siRNA

The cationic peptide of SEQ ID NO: 9 was dissolved in RNase, DNase Free & PCR certified Water (Tech&lnnovation, BWA-8000) at a concentration of 5 µg/µL. The siRNA of SEQ ID NO: 60 or SEQ ID NO: 61 was dissolved in RNase, DNase Free & PCR certified Water (Tech&lnnovation, BWA-8000) at a concentration of 1 µg/µL. The mixing ratio of siRNA solution and peptide of SEQ ID NO: 9 was 1:10, and 10 µL of siRNA was mixed with 12 µL (1:10) of peptide solution of SEQ ID NO: 9 in 2 µL increments, and left for at least 30 minutes.

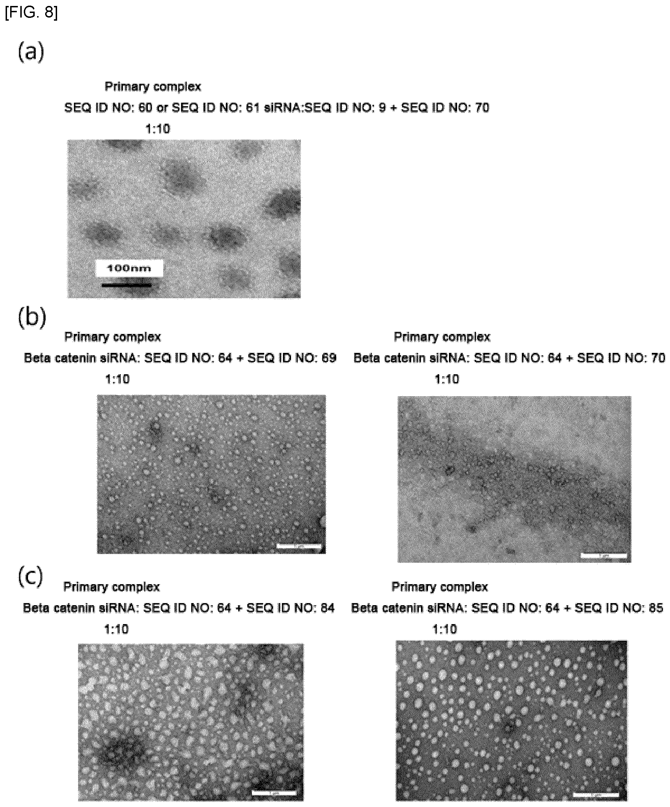

The peptide for penetrating the blood-brain barrier of SEQ ID NO: 70 was dissolved in RNase, DNase Free & PCR certified Water (Tech&lnnovation, BWA-8000) at a concentration of 1 µg/µL. The mixture of siRNA and peptide of SEQ ID NO: 9 was mixed with 12 µL of the peptide for penetrating the blood-brain barrier solution of SEQ ID NO: 70 in 2 µL increments and left for at least 30 minutes. The formed complex was confirmed using transmission electron microscopy (TEM, JEOL, JEM 1010) (FIG. 8(a)).

### Example 4-2: Preparation of a complex of beta catenin siRNA and a peptide for penetrating the blood brain barrier comprising a transferrin receptor binding peptide

The RNA binding peptide of SEQ ID NO: 64 was dissolved in RNase, DNase Free & PCR certified Water (Tech&lnnovation, BWA-8000) at a concentration of 5 µg/µL. Beta catenin siRNA (Dhamacon, #J-040628-05) was dissolved in RNase, DNase Free & PCR certified Water (Tech&lnnovation, BWA-8000) at a concentration of 1 µg/µL. The mixing ratio of siRNA solution and RNA binding peptide was 1:10, and 10 µL of siRNA was mixed with 12 µL (1:10) of RNA binding peptide solution in 1 µL increments, and left for at least 30 minutes.

The peptide for penetrating the blood-brain barrier of SEQ ID NO: 70 was dissolved in RNase, DNase Free & PCR certified Water (Tech&lnnovation, BWA-8000) at a concentration of 1 µg/µL. The mixture of beta catenin siRNA and RNA binding peptide of SEQ ID NO: 64 was mixed with 12 µL of peptide for penetrating the blood-brain barrier solution of SEQ ID NO: 70 in 1 µL increments and left for at least 30 minutes. The formed complex was confirmed using transmission electron microscopy (TEM, JEOL, JEM 1010) (FIG. 8(b)).

### Example 4-3: Preparation of a complex of peptide for penetrating the blood-brain barrier comprising beta catenin siRNA and LRP-binding peptide

The peptide of SEQ ID NO: 64 was dissolved in RNase, DNase Free & PCR certified Water (Tech&lnnovation, BWA-8000) at a concentration of 5 µg/µL. The siRNA of SEQ ID NO: 60 or SEQ ID NO: 61 was dissolved in RNase, DNase Free & PCR certified Water (Tech&lnnovation, BWA-8000) at a concentration of 1 µg/µL. The mixing ratio of siRNA solution and peptide carrier was 1:10, and 10 µL of siRNA was mixed with 12 µL (1:10) of peptide solution of SEQ ID NO: 64 was mixed in 1 µL increments, and left for at least 30 min. The peptide for penetrating the blood-brain barrier of SEQ ID NO: 84 was dissolved in RNase, DNase Free & PCR certified Water (Tech&lnnovation, BWA-8000) at a concentration of 1 µg/µL. The mixture of siRNA and peptide of SEQ ID NO: 64 was mixed with 12 µL of the peptide for penetrating the blood-brain barrier solution of SEQ ID NO: 84 in 2 µL increments and left for at least 30 minutes. The formed complex was confirmed using transmission electron microscopy (TEM, JEOL, JEM 1010) (FIG. 8(c)).

FIG. 8 shows the complex of the peptide for penetrating the blood-brain barrier and siRNA by transmission electron microscopy, confirming that each prepared complex formed particles with a size of 100 nm.

### Experimental Example 4: Verification of permeability in an in vitro BBB model

Using siRNA of SEQ ID NO: 60 or SEQ ID NO: 61 labeled with Alexa Fluor^{™} 488 (Invitrogen), a complex of peptide for penetrating the blood-brain barrier therewith was prepared in the same manner as in Example 4-1, and the permeability was evaluated using an in vitro BBB model. As a control, Lipofectamine^{™} 2000 (Invitrogen) was reacted with siRNA of SEQ ID NO: 60 or SEQ ID NO: 61 labeled with Alexa Fluor^{™} 488 (Invitrogen) according to the manufacturer's method. Immortalized mouse brain endothelial cells (bEnd.3, CRL-2299, ATCC) were seeded at 5×10⁴/well in the upper chamber of culture inserts (corning) and placed in 24 wells. In the lower chamber, 800 µL Dulbecco's modified Eagle's medium (DMEM) was added. The medium was changed every 2 days. Trans-endothelial electrical resistance (TEER) instrument (World Precision Instruments, Inc., Sarasota) was used to monitor the density of the cell layer, and the experiment was performed when the TEER value was 200 Ω cm² or more. The medium was replaced with DMEM without FBS, and the complex of the peptide for penetrating the blood-brain barrier of SEQ ID NO: 70 labeled with Cy5.5 and siRNA of SEQ ID NO: 60 or SEQ ID NO: 61, and the complex of Lipofectamine^{™} 2000 and siRNA of SEQ ID NO: 60 or SEQ ID NO: 61 were added to the upper chamber at a concentration of 200 nM each. The assembled transwell was incubated in a shaking incubator at 37°C, 50 rpm for 1, 4 and 8 hours. 500 µL was taken from the lower chamber (basolateral compartment) and the same volume of fresh medium was refilled. The TEER value was rechecked at the end of the experiment. The percentage of transported complex (transport ratio, %) was measured by fluorescence sensitivity using a fluorescence spectrophotometer (Thermo Scientific, USA). In Example 4-2 and Example 4-3, the permeability was also measured in the in vitro BBB model using the same method as above.

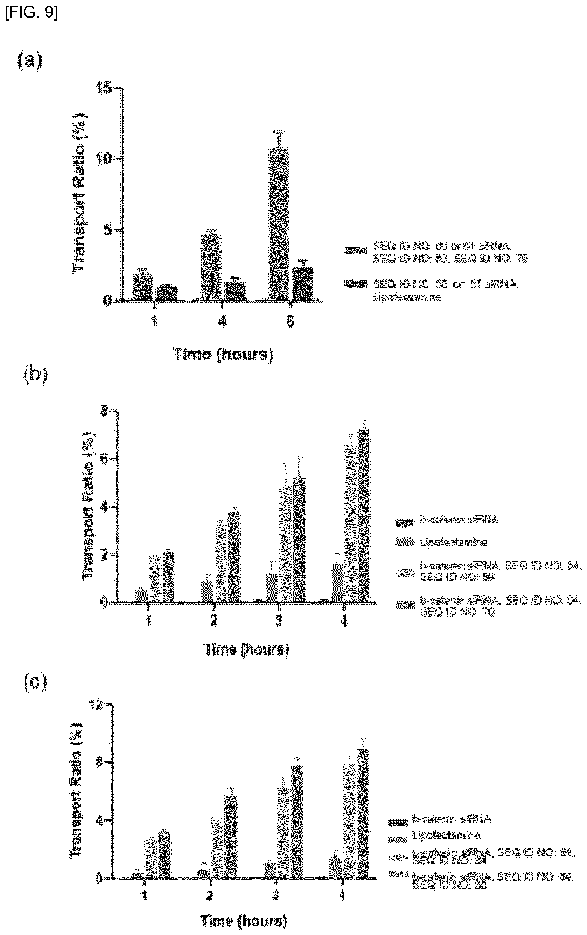

FIG. 9 shows the permeability results of the complex of the peptide for penetrating the blood-brain barrier and siRNA in an in vitro BBB model. The permeability of the complex of Example 4-1 increased with increasing time (FIG. 9(a)). Also, it was six times higher than that of the complex of lipofectamine and siRNA of SEQ ID NO: 60 or 61 after 8 hours of cell treatment. The complexes of Examples 4-2 and 4-3 were also found to be 8-10 times more permeable than Lipofectamine^{™} when measured up to 4 hours (FIG. 9(b) and FIG. 9(c)).

This means that the complexes of Example 4 effectively permeabilize the siRNA across the BBB.

### Experimental Example 5: Confirmation of brain delivery of a peptide for penetrating the blood-brain barrier and siRNA in normal animals

The siRNA of SEQ ID NO: 60 or SEQ ID NO: 61 labeled with Alexa Fluor^{™} 680 (Invitrogen) was used to prepare a complex with peptide for penetrating the blood-brain barrier in the same way as in Example 4-1.

The complex of Example 4-1 and siRNA of SEQ ID NO: 60 or SEQ ID NO: 61 are intravenously administered, respectively, at 150 µg/kg, to hairless mouse (SKH-1, female, 6 weeks old, OrientBio), and the brain was harvested 5 hours later and fluorescence was observed by confocal microscopy (Carl Zeiss LSM700).

Beta catenin siRNA labeled with Alexa Fluor^{™} 488 (Invitrogen) was used to prepare a complex with peptide for penetrating the blood-brain barrier in the same manner as in Examples 4-2 and 4-3.

The complex of Example 4-2 or 4-3 and beta catenin siRNA at 1 mg/kg are injected intravenously, respectively, at 1 mg/kg, to hairless mouse (SKH-1, female, 6 weeks old, OrientBio), and the brain was harvested 1 hour later and fluorescence was observed by confocal microscopy (Carl Zeiss LSM700).

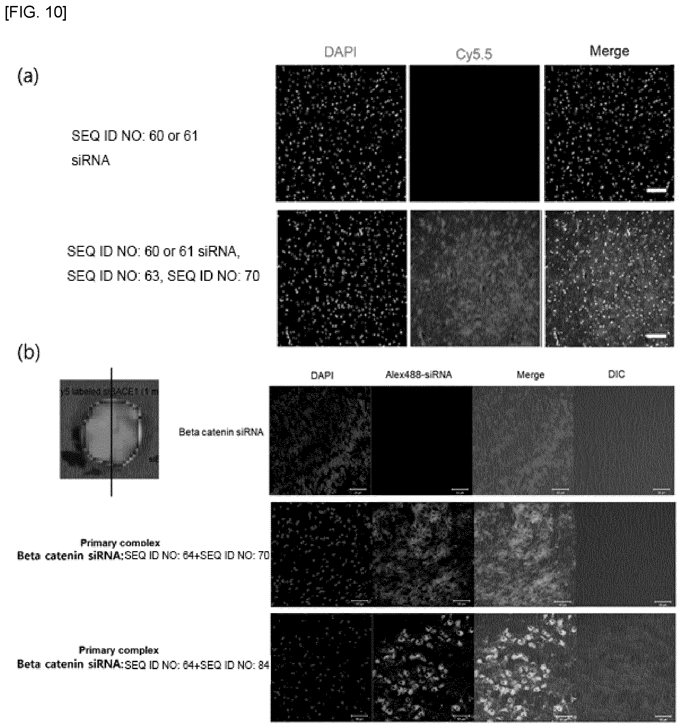

FIG. 10 shows the results of observing the complex of peptide for penetrating the blood-brain barrier and siRNA permeabilized into the brain parenchyma. It can be seen that the complex of Example 4-1 effectively delivers siRNA of SEQ ID NO: 60 or SEQ ID NO: 61 into the brain (FIG. 10(a)), and the complexes of Examples 4-2 and 4-3 also effectively deliver beta catenin siRNA into the brain (FIG. 10(b)).

### Experimental Example 6: Confirmation of beta catenin gene suppression in brain tissue by a complex of peptide for penetrating the blood-brain barrier and beta catenin siRNA in normal animals

It was observed whether beta catenin siRNA delivered to brain tissue effectively reduced beta catenin mRNA.

Eight-week-old mouse (C57BU6, OrientBio) was purchased, and a complex was prepared in the same manner as in Example 4-2 and Example 4-3, and injected intravenously at doses of 2.5mg/kg and 10mg/kg (siRNA-based). 24 and 96 hours after injection, blood was removed from the brain tissue by three 20 ml perfusions with PBS. The cerebral cortex, cerebellum and mid brain were isolated, and beta catenin mRNA in the brain tissue was measured by qRT-PCR (Thermo Fisher - QuantStudio 3). PCT conditions and used primer sequences are shown in Table 2 and Table 3, respectively.

**[Table 2]**

| | PCR Conditions | | | |
|---|---|---|---|---|
| Condition | | | | |
| Process | | Cycle number | Temperature(°C) | Time |
| Initialization | | 1 | 95 | 10 min |
| Denaturation | | 40 | 95 | 15 sec |
| Annealing | | 40 | 60 | 60 sec |
| Extension | | N/A | 72 | 60 sec |

**[Table 3]**

| Primer sequence | | | |
|---|---|---|---|
| Name | Species | Primer sequence(5'->3') | Annealing Tem(°C) |
| GAPDH | Mouse | F-GTATGACTCCACTCACGGCAAA (SEQ ID NO: 86) | 58-60°C |
| | | R-GGTCTCGCTCCTGGAAGATG (SEQ ID NO: 87) | |
| Ctnnb1 | Mouse | F-GATATTGACGGGCAGTATGCAA (SEQ ID NO: 88) | 58-60°C |
| | | R-AACTGCGTGGATGGGATGGGATCTG (SEQ ID NO: 89) | |

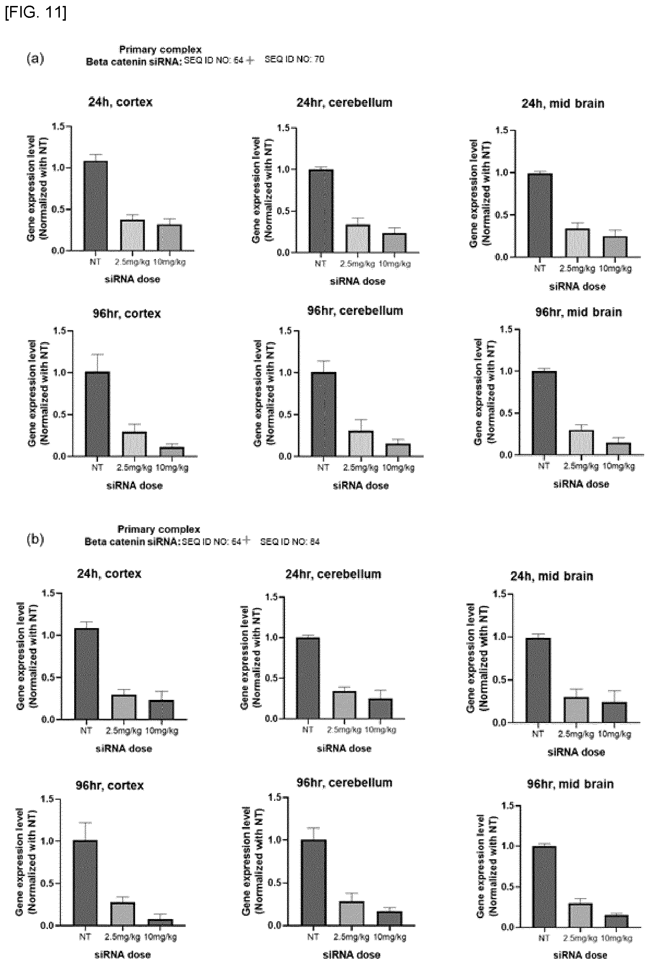

FIG. 11 shows the results of measuring beta catenin mRNA in the respective brain regions. Both the complexes of Example 4-2 (FIG. 11(a)) and 4-3 (FIG. 11(b)) effectively reduced beta catenin mRNA. Beta catenin mRNA was reduced more at 96 hours than at 24 hours after injection, and beta catenin mRNA levels decreased with increasing doses. Furthermore, the similar decrease in beta catenin mRNA levels in different regions of the brain suggests that the complex was delivered to each brain region.

### Industrial availability

The peptide for penetrating the blood-brain barrier of the present invention in which a cell-permeable peptide is linked to the brain endothelial cell surface protein-binding peptide passes through the cell membrane of the brain endothelial cell with excellent efficiency, and the peptide-drug complex in which the peptide for penetrating the blood-brain barrier is combined with a drug such as an antibody, a protein or a therapeutic nucleic acid has the advantage of effectively delivering the drug into the brain parenchyma and maximizing the therapeutic effect of brain diseases and brain tumors.

While the foregoing has described in detail certain aspects of the present invention, it would be apparent to one of ordinary skill in the art that these specific descriptions are merely preferred embodiments and are not intended to limit the scope of the present invention. Accordingly, the substantial scope of the present invention is defined by the appended claims and their equivalents.

## Claims

1. A peptide for penetrating the blood-brain barrier (BBB), wherein a brain endothelial cell surface protein-binding peptide is linked to a cell-permeable peptide.

2. The peptide for penetrating the blood-brain barrier according to claim 1, wherein the brain endothelial cell surface protein is a transferrin receptor or a low density lipoprotein receptor-related protein (LRP) receptor.

3. The peptide for penetrating the blood-brain barrier according to claim 2, wherein the transferrin receptor binding peptide is represented by an amino acid sequence of any one of SEQ ID NO: 1 to SEQ ID NO: 4.

4. The peptide for penetrating the blood-brain barrier according to claim 2, wherein the low density lipoprotein receptor-related protein receptor binding peptide is represented by an amino acid sequence of SEQ ID NO: 5 or SEQ ID NO: 6.

5. The peptide for penetrating the blood-brain barrier according to claim 1, wherein the cell-permeable peptide is represented by an amino acid sequence of any one of SEQ ID NO: 7 to SEQ ID NO: 10.

6. The peptide for penetrating the blood-brain barrier according to claim 1, wherein the peptide for penetrating the blood-brain barrier is represented by an amino acid sequence of any one of SEQ ID NO: 11 to SEQ ID NO: 58.

7. The peptide for penetrating the blood-brain barrier according to claim 1, wherein the brain endothelial cell surface protein-binding peptide and the cell-permeable peptide are linked via a spacer comprising 2 to 10 amino acids G and 1 to 5 amino acids S.

8. A peptide-drug complex for penetrating the blood-brain barrier, wherein a drug is conjugated to the peptide for penetrating the blood-brain barrier of any one of claims 1 to 7.

9. The peptide-drug complex for penetrating the blood-brain barrier according to claim 8, wherein the drug is an antibody, protein, or therapeutic nucleic acid.

10. The peptide-drug complex for penetrating the blood-brain barrier according to claim 8, wherein the peptide for penetrating the blood-brain barrier and the drug are connected by a linker.

11. The peptide-drug complex for penetrating the blood-brain barrier according to claim 10, wherein the drug is an antibody, wherein the linker is CGGGG.

12. The peptide-drug complex for penetrating the blood-brain barrier according to claim 9, wherein the antibody is represented by an amino acid sequence of SEQ ID NO: 59.

13. The peptide-drug complex for penetrating the blood-brain barrier according to claim 9, wherein the protein is selected from the group consisting of brain-derived neurotrophic factor (BDNF), nerve growth factor (NGF), neurotrophin-4/5, fibroblast growth factor (FGF)-2 and other FGFs, neurotrophin (NT)-3, erythropoietin (EPO), epidermal growth factor (EGF), transforming growth factor (TGF)-alpha, TGF-beta, vascular endothelial growth factor (VEGF), glial cell-inducing neurotrophic factor (GDNF), neurturin, platelet-derived growth factor (PDGF), herregulin, neuregulin, artemisin, persepsin, interleukin, glial cell line-induced neurotrophic factor (GFR), granulocyte-colony stimulating factor (CSF), and stem cell factor (SCF).

14. The peptide-drug complex for penetrating the blood-brain barrier according to claim 9, wherein the therapeutic nucleic acid is selected from the group consisting of DNA, siRNA, miRNA, and mRNA.

15. The peptide-drug complex for penetrating the blood-brain barrier according to claim 14, wherein the siRNA is represented by SEQ ID NO: 60 or SEQ ID NO: 61.

16. The peptide-drug complex for penetrating the blood-brain barrier according to claim 9, wherein the therapeutic nucleic acid is reacted with a cationic peptide, cationic polymer, or RNA binding peptide to prepare a primary complex, and then the primary complex is reacted with the peptide for penetrating the blood-brain barrier of claim 1 linked to an anionic amino acid or anionic polymer; and a spacer to form a nanoparticle.

17. The peptide-drug complex for penetrating the blood-brain barrier according to claim 16, wherein the cationic peptide is represented by the amino acid sequence of SEQ ID NO: 9, the cationic polymer is poly-L-lysine, polyethylene imine, or chitosan, and the RNA binding peptide is represented by the amino acid sequence of any one of SEQ ID NO: 62 to SEQ ID NO: 67, the anionic amino acid is glutamic acid or aspartic acid, the anionic polymer is heparin or hyaluronic acid, and the spacer is 5 to 10 glycines.

18. A nucleic acid encoding the peptide-drug complex for penetrating the blood-brain barrier of claim 8.

19. A recombinant vector into which the nucleic acid of claim 18 is introduced.

20. A recombinant cell into which the nucleic acid of claim 18 or the recombinant vector of claim 19 is introduced.

21. A method of preparing a peptide-drug complex for penetrating the blood-brain barrier according to claim 8, comprising the following steps:
(a) culturing the recombinant cell of claim 20 to express the peptide-drug complex for penetrating the blood-brain barrier; and
(B) recovering the expressed peptide-drug complex for penetrating the blood-brain barrier.

22. A pharmaceutical composition for treating or preventing brain diseases or brain tumors, comprising the peptide-drug complex for penetrating the blood-brain barrier according to claim 8.

23. The pharmaceutical composition according to claim 22, wherein the brain disease is selected from the group consisting of encephalitis, Parkinson's disease, epilepsy, Huntington's disease, Alzheimer's disease, Lou Gehrig's disease, Pick's disease, epilepsy, Creutzfeldt-Jakob disease, progressive supranuclear palsy, spinocerebellar degeneration, cerebellar atrophy, multiple sclerosis, senile dementia caused by the loss of nerve cells, stroke, amnesia, depression, and post-traumatic stress disorder.

24. The pharmaceutical composition according to claim 22, wherein the brain tumor is selected from the group consisting of glioma, glioblastoma multiforme, meningioma, astrocytoma, acoustic neuroma, chordoma, rare glioblastoma, medulloblastoma, ganglioneuroma, schwannoma, neurofibroma, neuroblastoma, and epidural, intramedullary or intrathecal tumors.

25. The pharmaceutical composition according to claim 22, wherein a single dose of the peptide-drug complex for penetrating the blood-brain barrier is 1 µg/kg to 100 mg/kg.
